# EUROPÄISCHE PATENTSCHRIFT

(11) **EP 2 588 147 B1**
(45) Veröffentlichungstag und Bekanntmachung des Hinweises auf die Patenterteilung: **30.04.2014**
(21) Anmeldenummer: 11711466.0
(22) Anmeldetag: 24.03.2011
(51) Int. Cl.: A61L 2/20, B67B 3/00

(54) **VORRICHTUNG ZUM STERILISIEREN VON VERSCHLÜSSEN FÜR BEHÄLTER**
DEVICE FOR STERILIZING CLOSURES FOR CONTAINERS
DISPOSITIF DESTINÉ À STÉRILISER DES ÉLÉMENTS DE FERMETURE POUR DES RÉCIPIENTS

(30) Priorität: 29.06.2010 DE 102010025541
(43) Veröffentlichungstag der Anmeldung: 08.05.2013
(73) Patentinhaber: KHS GmbH, 44143 Dortmund (DE)
(72) Erfinder: DRENGUIS, Alfred, 21039 Börnsen (DE)
(86) Internationale Anmeldenummer: PCT/EP2011/001465
(87) Internationale Veröffentlichungsnummer: WO 2012/000573

(56) Entgegenhaltungen:
- EP-A1- 1 749 747
- WO-A1-00/46142
- WO-A2-2010/031464
- JP-A- 2003 128 023

## Beschreibung

Die Erfindung bezieht sich auf eine Vorrichtung zum Sterilisieren von Verschlüssen gemäß Oberbegriff Patentanspruch 1.

WO2010031464 und EP1749747 offenbaren jeweils eine Vorrichtung zum Sterilisieren von Kappen oder dergleichen Verschlüssen zum Verschließen von Flaschen oder dergleichen Behältern, mit einem die Verschlüsse in einer Transportrichtung durch eine Behandlungsstrecke mit mehreren in der Transportrichtung aufeinander folgenden Behandlungsstationen transportierenden Transportsystem.

Beispielsweise bei Anlagen der Getränkeindustrie ist es üblich und bekannt, die zum Verschließen von gefüllten Flaschen oder anderen Behältern verwendeten Verschlüsse, insbesondere kappenartige Verschlüsse, wie Schraubverschlüsse, Flachkappen, Kronkorken usw. vor ihrer Verwendung, d.h. vor dem Aufbringen auf den jeweiligen Behälter zu sterilisieren, und zwar u.a. unter Verwendung von Sterilisiermitteln, die aus H2O2-Dampf (verdampfte wässrige WasserstoffperoxidLösung mit ausreichend hoher H2O2-Konzentation) oder aus einem mit H2O2-Dampf angereicherten gas- und/oder dampfförmigen Trägermedium bestehen. Um eine wirksame Sterilisation mit hoher Entkeimungsrate zu erreichen, werden die Verschlüsse vor der Beaufschlagung mit dem Sterilisiermittel vorerwärmt, beispielsweise auf eine Temperatur im Bereich zwischen 50°C und 85°C und dann nach der Behandlung bzw. Beaufschlagung mit dem gas- und/oder dampfförmigen Sterilisiermittel unter Verwendung eines vorzugsweise erwärmten sterilen gas- und/oder dampfförmigen Mediums, beispielsweise unter Verwendung von vorzugsweise erwärmter steriler Luft getrocknet, sodass die sterilisierten Verschlüsse dann anschließend zum Verschließen von Behältern verwendet bzw. hierfür einer Verschließmaschine zugeführt werden können.

Aufgabe der Erfindung ist es, eine Vorrichtung aufzuzeigen, die bei hoher Qualität der Sterilisation oder Entkeimungsrate auch eine hohe Leistung (Anzahl der sterilisierten Verschlüsse je Zeiteinheit) ermöglicht. Zur Lösung dieser Aufgabe ist eine Vorrichtung entsprechend dem Patentanspruch 1 ausgebildet.

Besondere Vorteile der erfindungsgemäßen Vorrichtung bestehen u.a. darin, dass die Vorrichtung trotz hoher Leistung sehr kompakt und mit kleinem Bauraum oder Bauvolumen realisierbar ist, und dass gleichbleibend bzw. reproduzierbar hohe Entkeimungsraten bei der Sterilisation erreicht werden. Die erfindungsgemäße Vorrichtung eignet sich für Verschlüsse unterschiedlichster Art, insbesondere auch für kappenartige Verschlüsse, wie Schraubverschlüsse, Flachkappen, Kronkorken usw.

Bevorzugt sind bei der erfindungsgemäßen Vorrichtung die dortigen Behandlungszonen jeweils mit einem leichten Überdruck eines sterilen gas- und/oder dampfförmigen Mediums beaufschlagt, sodass ein Eindringen von Umgebungsluft und von mit dieser mitgeführten Keimen in die Behandlungszonen wirksam vermieden ist, insbesondere auch im Bereich von Verschlussein- und Auslässen.

Die Erfindung wird im Folgenden anhand der Figuren an einem Ausführungsbeispiel näher erläutert. Es zeigen:

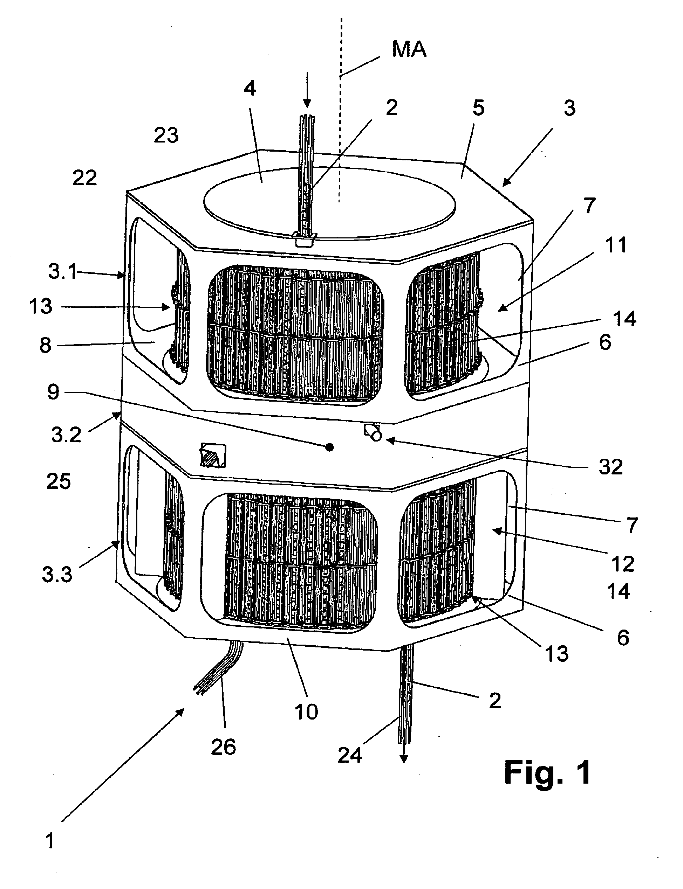

- Fig. 1: in perspektivischer Darstellung und in unterschiedlichen Ansichten eine Vorrichtung gemäß der Erfindung zum Sterilisieren von Verschlüssen, die zum Verschließen von Flaschen oder dergleichen Behältern bestimmt sind;

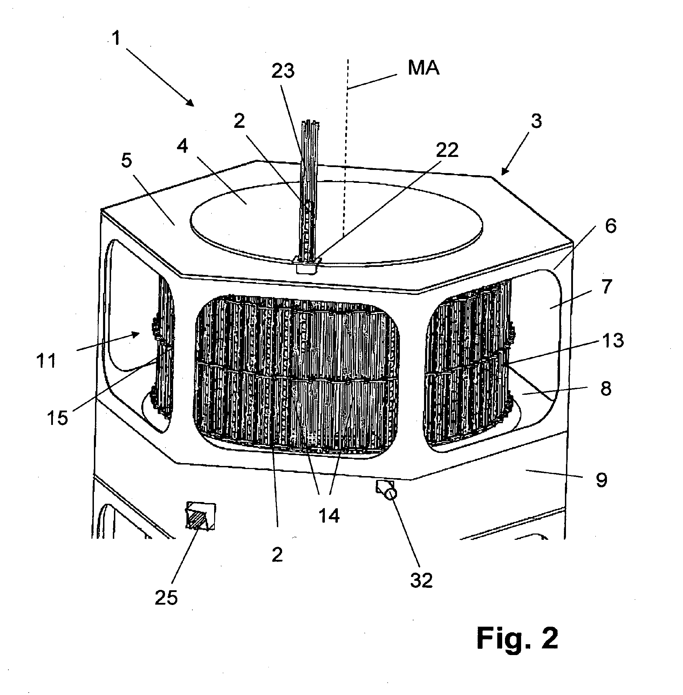

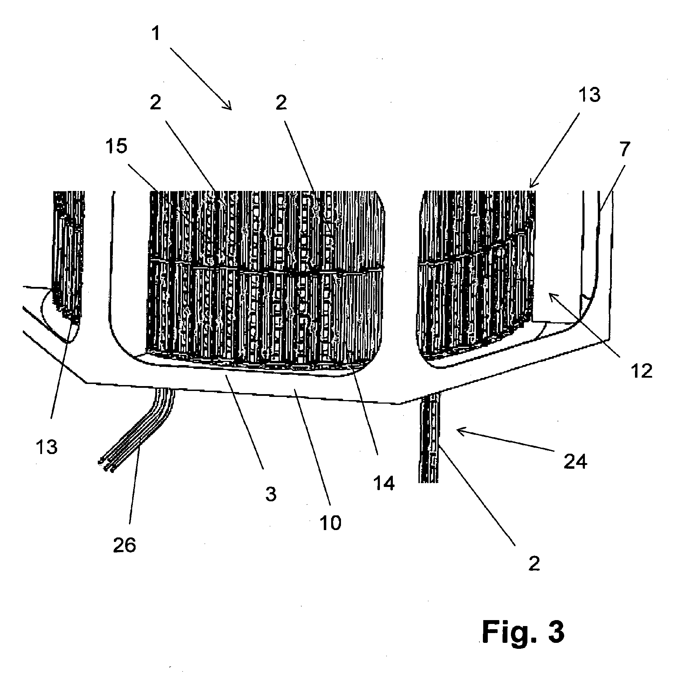

- Fig. 2 und 3: in vergrößerter perspektivischer Ansicht Ausschnitte der Darstellung der Figur 1;

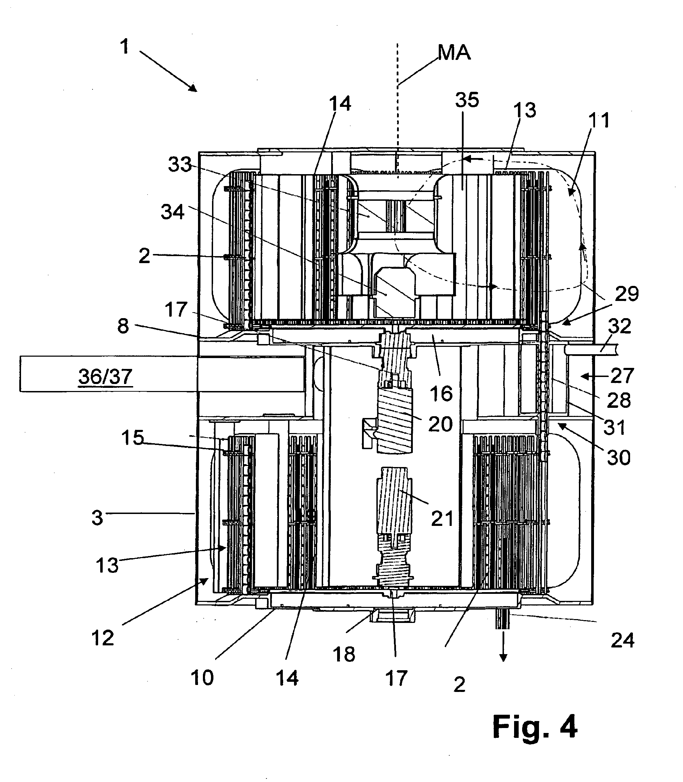

- Fig. 4: in vereinfachter Darstellung einen Vertikalschnitt der Vorrichtung der Figur 1;

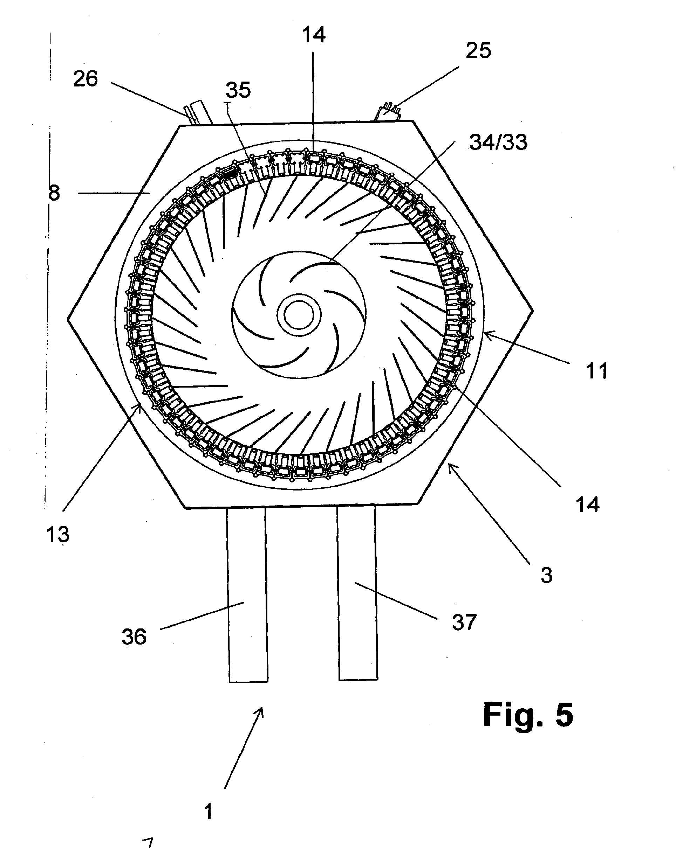

- Fig. 5: in vereinfachter Darstellung einen Horizontalschnitt durch eine obere als Vorheizzone dienenden Behandlungszone der Vorrichtung der Figuren 1;
- Fig. 6: in vereinfachter Teildarstellung und in Seitenansicht zwei der an einem Rotor der Vorrichtung gebildeten Verschlussaufnahmen.

Die in den Figuren allgemein mit 1 bezeichnete Vorrichtung dient zum Sterilisieren von Verschlüssen 2, beispielsweise von Verschlüssen in Form von Kappen, Schraubkappen, Flachkappen oder Kronkorken usw., die zum Verschließen von nicht dargestellten Behältern z.B. in Form von Flaschen verwendet werden. Die Vorrichtung 1 bildet hierfür bevorzugt ein einer Verschließmaschine zum Verschließen der Behälter vorgeschaltetes Aggregat, aus dem die sterilisierten Verschlüsse 2 unter sterilen bzw. keimfreien Bedingungen der Verschließmaschine zugeführt werden.

Die Vorrichtung 1 umfasst u.a. ein Vorrichtungsgehäuse 3, welches in Draufsicht eine eine vertikale Maschinenachse MA umschließende polygonale Formgebung aufweist, d.h. bei der dargestellten Ausführungsform sechseckförmig ausgebildet ist.

Der Innenraum des Gehäuses 3 ist mit Ausnahme von Ein- und Auslässen für die Verschlüsse 2 gegenüber der Umgebung dicht verschlossen.

Bei der dargestellten Ausführungsform besteht das Gehäuse 3 aus drei in Achsrichtung der Maschinenachse MA aneinander anschließenden Gehäuseabschnitten, nämlich
- aus einem oberen Gehäuseabschnitt 3.1, welcher im Wesentlichen die mit einem Inspektionsdeckel 4 versehene obere sechseckförmige Gehäusewand 5, eine Umfangswand 6, die mit großformatigen, aber jeweils durch eine Verglasung dicht verschlossenen Inspektionsfenster 7 versehen ist, sowie einen Zwischenboden 8 umfasst,
- aus einem anschließenden Gehäuseabschnitt 3.2 mit geschlossener Umfangswand 9, sowie
- aus dem anschließenden Gehäuseabschnitt 3.3, welcher im Wesentlichen die untere sechseckförmige Gehäusewand 10 und Umfangswand 6 mit den großformatigen, durch eine Verglasung dicht verschlossenen Inspektionsfenster 7 umfasst.

Hierdurch ergibt sich ein modulartiger, die Herstellung vereinfachender Aufbau zumindest des Gehäuses 3.

Durch den Zwischenboden 8 ist der Innenraum des Gehäuses 3 in eine obere im Gehäuseabschnitt 3.1 ausgebildete Behandlungszone 11 und in eine untere, von der Behandlungszone 11 getrennte sowie in den Gehäuseabschnitten 3.2 und 3.3 ausgebildete Behandlungszone 12 unterteilt. In beiden Behandlungszonen 11 und 12 ist jeweils ein die gemeinsame vertikale Maschinenachse MA umlaufend antreibbarer Rotor 13 vorgesehen. Bei der dargestellten Ausführungsform sind die beiden Rotoren 13, die jeweils Teil eines Transportsystem für die Verschlüsse 2 sind, identisch ausgeführt, und zwar jeweils als kreiszylinderförmige Hohltrommel mit einem Trommelmantel mit käfigartiger Struktur, die von einer Vielzahl von Verschlussaufnahmen 14 zur Aufnahme jeweils einer Vielzahl von Verschlüssen 2 gebildet ist. Die Verschlussaufnahmen 14, die mit ihrer Längserstreckung jeweils parallel oder im Wesentlichen parallel zur Maschinenachse MA orientiert und an ihrem oberen Ende zum Einbringen der Verschlüsse 2 und an ihrem unteren Ende zum Ausbringen der Verschlüsse 2 offen sind, bilden in ihrer Gesamtheit die käfigartige Struktur bzw. den käfigartigen Mantel des jeweiligen Rotors 13 und sind hierfür am Umfang des Rotors 13 in gleichmäßigen Winkel- oder Teilungsabständen um die Maschinenachse MA verteilt angeordnet. Durch die Maschinenachse MA konzentrisch umschließende und in Richtung dieser Achse gegeneinander versetzte ringartige Halterungen 15 sind die Verschlussaufnahmen 14 miteinander zu der gitterartigen Struktur des jeweiligen Rotors 13 verbunden. Im Bereich ihrer unteren Enden sind die Verschlussaufnahmen 14 jedes Rotors 13 an einem achsgleich mit der Maschinenachse MA angeordneten kreisscheibenförmigen Tragelement 16 befestigt, und zwar derart, dass die Verschlussaufnahmen 14 jeweils über die Umfangsfläche des Tragelementes 16 vorstehen.

Die Verschlussaufnahmen 14 sind ebenfalls gitterartig ausgeführt und bestehen jeweils aus mehreren voneinander beabstandeten und parallel zur Maschinenachse MA orientierten stangen- oder stabförmigen Verschlussführungsschienen, die zwischen sich einen Aufnahmeraum zur Aufnahme einer Vielzahl von Verschlüssen 2 bilden, und zwar derart, dass die Verschlüsse 2 in jeder Verschlussaufnahme 14 eine sich in eine Achsrichtung parallel zur Maschinenachse MA erstreckende einspurige Reihe bilden und in den Verschlussaufnahmen 14 möglichst weitestgehend freiliegen, d.h. jeweils nur an einem geringen Teil ihrer Oberfläche von den Verschlussführungsschienen 14 abgedeckt sind. Weiterhin sind die Verschlussaufnahmen 14 bei der dargestellten Ausführungsform so ausgeführt, dass die Verschlüsse 2 in den Verschlussaufnahmen 14 in Bezug auf die Maschinenachse MA eine vorgegebene Orientierung aufweisen, und zwar in der Form, dass sie mit ihrer Verschluss- oder Kappenachse radial zur Maschinenachse MA und beispielsweise mit ihrer offenen Kappenseite radial nach außen orientiert sind.

Mit dem Tragelement 16 bzw. mit einer mit dem Tragelement 16 verbundenen und Achsgleich mit der Maschinenachse MA angeordneten Welle 17 ist der obere Rotor 13 in der Behandlungszone 11 in einem Lager 18 des Zwischenbodens 8 und der untere Rotor 13 in der Behandlungszone 12 an einem an der unteren Gehäusewand 10 vorgesehenen Lager 19 um die Maschinenachse MA drehbar gelagert. Jedem Rotor 13 ist ein eigenständiger Antrieb mit Getriebe und eigenständigem Antriebsmotor (Elektromotor) zugeordnet, und zwar dem oberen Rotor 13 der Antrieb 20 und dem unteren Rotor 13 der Antrieb 21.

Im oberen Bereich der Behandlungszone 11 ist über der Bewegungsbahn der Verschlussaufnahmen 14 eine Verschlussaufgabe 22 vorgesehen, der die zu sterilisierenden Verschlüsse 2 über eine Förderstrecke 23 zugeführt wird, in welcher die Verschlüsse 2 bereits die ihrer Orientierung in den Verschlussaufnahmen 14 entsprechende Orientierung aufweisen. Im Bereich der Unterseite des Gehäuses 3 ist unterhalb der Bewegungsbahn der Verschlussaufnahmen 14 eine Verschlussabgabe vorgesehen, die im Wesentlichen von dem Einlass oder von dem offenen Ende einer Förderstrecke 24 gebildet ist, die mit diesem Ende durch die untere Gehäusewand 10 hindurch in die untere Behandlungszone 12 hinein reicht und mit ihrem offenen Ende unterhalb der Bewegungsbahn der Verschlussaufnahmen 14 des unteren Rotors 13 angeordnet ist. Über die Förderstrecke 24 werden die sterilisierten Verschlüsse 2 der nicht dargestellten Verschließmaschine keimfrei zugeführt.

Zusätzlich zu der von der Förderstrecke 24 gebildeten Verschlussabgabe sind zwei jeweils von einer Verschlussführung 25 bzw. 26 gebildete zusätzliche Verschlussabgaben zum Leerfahren der Vorrichtung 1 und/oder der Rotoren 13, d.h. zum Entfernen von Verschlüssen 2 aus der Vorrichtung 1 und/oder aus den Rotoren 13 bzw. aus deren Verschlussaufnahmen 14 vorgesehen, und zwar beispielsweise bei Störungen in einer die Vorrichtung 1 aufweisenden Anlage, bei einem Formatwechsel, d.h. beim Umstellen von einer Verschlussart auf eine andere Verschlussart usw. Die aus dem Gehäuse 3 herausgeführten Verschlussführungen 25 und 26 enden jeweils in den Behandlungszonen 11 bzw. 12 unterhalb der Bewegungsbahn der Verschlussaufnahmen 14. Durch entsprechende, nicht dargestellte Steuermittel sind die dortigen Einlässe der Verschlussführungen 25 und 26 derart steuerbar, dass im normalen Betrieb der Vorrichtung 1 Verschlüsse 2 nicht in die Verschlussführungen 24 und 26 gelangen, sondern lediglich beim Leerfahren der Vorrichtung 1 und/oder der Rotoren 13.

Bei der dargestellten Ausführungsform sind die Förderstrecken 23 und 24, aber auch die Verschlussführungen 25 und 26 jeweils von mehreren, die Verschlüsse zwischen sich aufnehmenden und führenden Führungsschienen gebildet. Weiterhin weisen bei der dargestellten Ausführungsform zumindest die Förderstrecken 23 und 24 wenigstens in der Nähe der Vorrichtung 1 jeweils einen vertikalen Verlauf auf, sodass die Verschlüsse 2 in diesen Förderstrecken ebenso wie in Verschlussaufnahmen 14 allein schon aufgrund ihrer Schwerkraft bewegt bzw. gefördert werden. Um ein erneutes Verkeimen der sterilisierten Verschlüsse 2 auf der Förderstrecke 24 zu vermeiden, ist diese in einer nicht dargestellten Ummantelung oder Einhausung aufgenommen, die bevorzugt mit einem Überdruck eines sterilen gas- und/oder dampfförmigen Mediums, beispielsweise mit dem Überdruck steriler Luft beaufschlagt ist.

Im Inneren des Gehäuses 3 ist zwischen der Unterseite des oberen Rotors 13 und der Oberseite des unteren Rotors 13 eine Behandlungsstrecke oder -zone 27 gebildet, und zwar u.a. bestehend aus einer gitterartigen, sich parallel zur Maschinenachse MA erstreckenden Verschlussführung 28, die für eine Aufnahme einer Vielzahl von Verschlüssen 2 in einer einspurigen Reihe ausgebildet ist, in der die Verschlüsse 2 bezogen auf die Maschinenachse MA in gleicher Weise orientiert sind wie in den Verschlussaufnahmen 14. Die durch den Zwischenboden 8 hindurchgeführte Verschlussführung 28 ist mit ihren oberen offenen Ende an einer Verschlussübergabeposition 29 unterhalb der Bewegungsbahn der Verschlussaufnahmen 14 des oberen Rotors 13 und mit ihrem unteren offenen Ende an einer Verschlussübergabeposition 30 oberhalb der Bewegungsbahn der Verschlussaufnahme 14 des unteren Rotors 13 angeordnet. Die Verschlussführung 28 ist zumindest über den größeren Teil ihrer Länge in einer Kammer 31 aufgenommen, die sich unterhalb des Zwischenbodens 8 in der Behandlungszone 12 befindet und in der die Verschlüsse 2 mit dem heißen Sterilisiermittel behandelt bzw. beaufschlagt werden, das von H2O2-Dampf gebildet ist oder H2O2-Dampf in einem gas- und/oder dampfförmigen Trägermedium enthält und das in die Kammer 31 über einen Einlass 32 eingeleitet wird.

Die obere Behandlungszone 11 dient zum Vorwärmen der Verschlüsse 2 auf eine gewünschte oder vorgewählte Vorwärmtemperatur, beispielsweise im Bereich zwischen 50°C und 85°C. Hierfür ist dort innerhalb des Rotors 13 eine Heizung 33 mit Umwälz- oder Umluftgebläse 34 vorgesehen, welchem eine Vielzahl von lamellenartigen Führungs- oder Leitblechen 35 zugeordnet ist und welches so angetrieben ist, dass sich innerhalb der Behandlungszone 11 ein Strom eines heißen gas- und/oder dampfförmigen Mediums, beispielsweise ein Heißluftstrom ergibt, und zwar mit einer Strömungsrichtung vom Umluftgebläse 34 durch den Rotor 13 bzw. durch die Verschlussaufnahmen 14 hindurch radial nach außen (schwerpunktmäßig im unteren Bereich der Behandlungszone 11) und dann wieder zumindest teilweise durch den Rotor 13 bzw. durch die Verschlussaufnahmen 14 hindurch zurück in Richtung Rotormitte (schwerpunktmäßig im oberen Bereich der Behandlungszone 11).

Die untere Behandlungszone 12 dient im Wesentlichen zum Trocknen der Verschlüsse 2 mit einem sterilen, bevorzugt erwärmten gas- und/oder dampfförmigen Medium, beispielsweise mit bevorzugt erwärmter Sterilluft. Das gas- und/oder dampfförmige Medium wird über einen Einlass 36 zugeführt und über einen Auslass 37 abgeführt. Die Ausbildung ist dabei so getroffen, dass ein direkter Strom zwischen dem Einlass 36 und dem Auslass 37 verhindert ist, und dass sich über Öffnungen im Zwischenboden 8 auch eine Strömung des sterilen Mediums aus der Behandlungszone 12 in die Behandlungszone 11 sowie an den Durchführungen zumindest der Förderstrecke 23 und der Verschlussführungen 25 und 26 eine Strömung des sterilen Mediums nach außen einstellt, um so ein Eindringen von Umgebungsluft in das Innere des Gehäuses 3 zu vermeiden.

Die Verschlussaufgabe 22 und die Übergabeposition 29 sind in einem Winkelabstand, der einem Vielfachen des Teilungsabstandes der Verschlussaufnahmen 14 am oberen Rotor 13 entspricht, so angeordnet, dass sich immer dann, wenn eine Verschlussaufnahme 14 des oberen Rotors 13 mit ihren oberen offenen Ende die Verschlussübergabe 22 erreicht hat, sich eine andere Verschlussaufnahme 14 dieses Rotors mit ihren unteren offenen Ende an der Übergabeposition 29 befindet. In gleicher Weise sind die untere Übergabeposition 30 und die von der Förderstrecke 24 gebildete Verschlussabgabe in einem Winkelabstand, der einem Vielfachen des Teilungsabstandes der Verschlussaufnahmen 14 am unteren Rotor 13 entspricht, so angeordnet, dass sich immer dann, wenn eine Verschlussaufnahme 14 des unteren Rotors 13 mit ihren oberen offenen Ende die Übergabeposition 30 erreicht hat, sich eine andere Verschlussaufnahme 14 dieses Rotors mit ihren unteren offenen Ende an der Verschlussabgabe befindet.

Bezogen auf die Drehrichtung des jeweiligen Rotors 13 sind die Verschlussaufgabe 22 und die Verschlussübergabeposition 29 und die Verschlussübergabeposition 30 und die von der Förderstrecke 24 gebildete Verschlussabgabe um möglichst einen großen Winkelbereich voneinander beabstandet, beispielsweise um einen Winkelbereich von wenigstens 330°.

Zumindest während des normalen Betriebes sind die beiden Rotoren 13 durch ihre Antriebe 20 und 21 gleichsinnig und synchron getaktet derart angetrieben, dass insbesondere dann, wenn sich eine Verschlussaufnahme 14 des oberen Rotors 13 an der Übergabeposition 29 befindet, an der Übergabeposition 30 eine leere Verschlussaufnahme 14 des unteren Rotors bereitsteht.

An der Verschlussaufgabe 22 wird in jeder Stillstandsphase der getakteten Drehbewegung des oberen Rotors 13 eine Verschlussaufnahme 14 dieses Rotors beispielsweise vollständig mit Verschlüssen 2 gefüllt, die in der Förderstrecke 23 als ausreichend großer Vorrat an Verschlüssen bereit gestellt werden. Mit dem umlaufenden oberen Rotor 13 werden dann die in der jeweiligen Verschlussaufnahme 14 aufgenommenen Verschlüsse 2 von der Verschlussaufgabe 22 durch die Behandlungszone 11 an die Verschlussübergabeposition 29 bewegt und dabei durch den von der Heizung 33 und dem Umluftgebläse 34 erzeugten heißen Strom erhitzt werden.

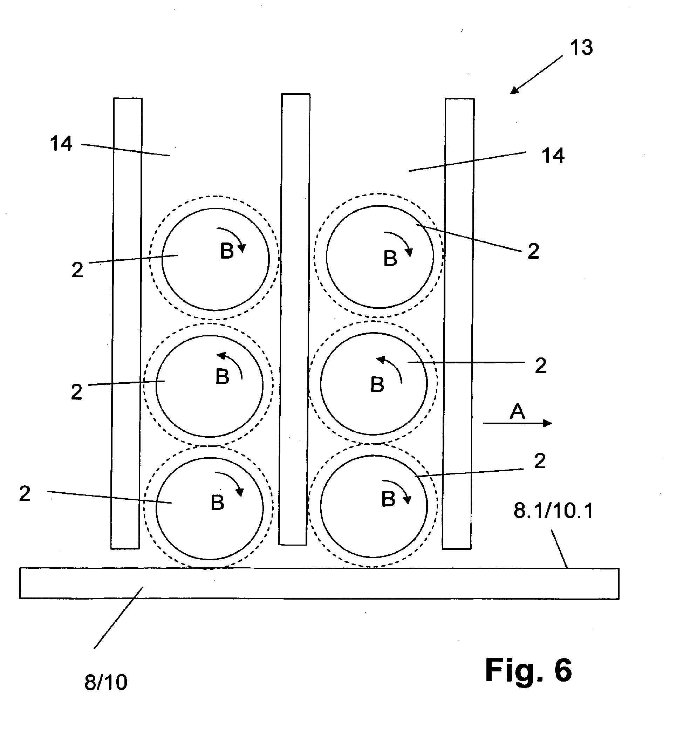

Wie in der Figur 6 dargestellt, wälzt sich bei der mit dem Pfeil A angedeuteten Drehbewegung des Rotors 13 der jeweils unterste in einer Verschlussaufnahme 14 angeordnete Verschluss 2 jeder Verschlussaufnahme 14 mit seiner Umfangsfläche reibradartig auf einer von dem Zwischenboden 8 gebildeten und mit dem Rotor 13 nicht mitbewegten Verschlussanlagefläche 8.1 ab, wodurch entsprechend dem Pfeil B eine Dreh- oder Abwälzbewegung für den untersten Verschluss 2 erzeugt wird, die sich von Verschluss zu Verschluss auf sämtliche in der jeweiligen Verschlussaufnahme 14 angeordneten und mit ihren Umfangsflächen gegen einander anliegenden Verschlüsse 2 überträgt, sodass sich sämtliche Verschlüsse 2 in jeder Verschlussaufnahme 14 entsprechend den Pfeilen B gegenläufig drehen und dadurch an ihrer gesamten Oberfläche mit dem Heißluftstrom beaufschlagt werden.

Immer dann, wenn eine Verschlussaufnahme 14 des oberen Rotors 13 die Verschlussübergabeposition 29 erreicht hat, fallen sämtliche Verschlüsse 2 dieser Aufnahme durch die Behandlungszone 27 bzw. durch die dortige Verschlussführung 28 in eine bereitstehende leere Verschlussaufnahme 14 des unteren Rotors 13. In der Behandlungszone 27 werden die Verschlüsse 2 mit dem heißen Sterilisiermittel beaufschlagt, welches ständig über den Anschluss 32 zugeführt wird.

Die in der jeweiligen Verschlussaufnahme 14 des unteren Rotors 13 aufgenommenen Verschlüsse 2 werden mit diesem Rotor 13 durch die Behandlungszone 12 bewegt und dabei durch den Strom des sterilen gas- und/oder dampfförmigen Mediums getrocknet. Entsprechend der Figur 6 wälzt sich wiederum bei der Drehbewegung (Pfeil A) des unteren Rotors 13 der jeweils unterste Verschluss 2 in jeder Verschlussaufnahme 14 mit seiner Umfangsfläche reibradartig auf einer von der Gehäusewand 10 gebildeten und mit dem Rotor 13 nicht mitbewegten Verschlussanlagefläche 10.1 ab, wodurch eine Dreh- oder Abwälzbewegung (Pfeil B) erzeugt wird, die sich von Verschluss zu Verschluss auf sämtliche in der jeweiligen Verschlussaufnahme 14 angeordneten und mit ihren Umfangsflächen gegen einander anliegenden Verschlüsse 2 überträgt, sodass sich diese gegenläufig drehen (Pfeile B) und dadurch an sämtlichen Oberflächenbereichen optimal getrocknet werden. An der Verschlussabgabe fallen dann sämtliche Verschlüsse der jeweiligen Verschlussaufnahme in die Förderstrecke 24 zur Weiterführung an den Verschließer.

Durch die getrennten Antriebe 20 und 21 sind verschiedene Betriebsweisen der Vorrichtung 1 möglich. So besteht beispielsweise die Möglichkeit, am Betriebsbeginn die Verschlussaufnahmen 14 des oberen Rotors vollständig mit Verschlüssen 2 zu füllen und den unteren Rotor 13 über seinen Antrieb 21 erst dann getaktet anzutreiben, wenn eine Verschlussreihe aus den oberen Rotor 13 in die Verschlussaufnahme 14 des unteren Rotors 13 eingebracht ist. Umgekehrt ist es vor einem Betriebsende auch möglich, den oberen Rotor 13 abzuschalten und den unteren Rotor unter Abgabe sämtlicher Verschlüsse an die Förderstrecke 24 leer zu fahren. Weiterhin besteht durch die getrennten Antriebe 20 und 21 die Möglichkeit, beispielsweise bei Störungen und/oder bei einem Formatwechsel die Rotoren 13 individuell leer zu fahren, d.h. die Verschlüsse jeweils beispielsweise auch über die Verschlussführungen 25 bzw. 26 auszubringen.

Vorstehend wurde davon ausgegangen, dass die Bewegung der Verschlüsse 2 innerhalb der Förderstrecken 23 und 24, der Verschlussführungen 24 und 26, der Verschlussführung und der Verschlussaufnahmen 14 jeweils durch Schwerkraft erfolgt. Grundsätzlich besteht auch die Möglichkeit, an diesen Elementen Transportdüsen für den Austritt eines sterilen gas- und/oder dampfförmigen Fördermediums, beispielsweise für den Austritt steriler Förderluft vorzusehen.

Weiterhin sind bevorzugt an den Behandlungszonen 11 und 12 sowie an der Behandlungsstrecke 27 nicht dargestellte Mittel zum Reinigen und/oder nicht dargestellte Messfühler zur Messung und/oder Überwachung des Zustandes der Behandlungszonen sowie an der Behandlungsstrecke 27 vorgesehen.

Weiterhin kann die Behandlungszone 27 auch wenigstens zwei vorzugsweise parallele Verschlussführungen 28 aufweisen, die dann so angeordnet sind, dass in jeder Stillstandsphase der getakteten Drehbewegung der Rotoren 13 jede Verschlussführung 28 mit ihrem Einlass oder mit ihrem oberen Ende dem Auslass oder dem unteren offenen Ende einer Verschlussaufnahme 14 des oberen Rotors 13 gegenüber liegt und mit ihrem Auslass oder mit ihrem unteren Ende dem Einlass oder dem oberen offenen Ende einer leeren Verschlussaufnahme 14 des unteren Rotors 13 gegenüber liegt. Die Verschlussaufgabe 22 und die von der Förderstrecke 24 gebildete Verschlussabgabe sind dabei dann zum gleichzeitigen Füllen bzw. Entleeren mehrerer Verschlussaufnahmen 14 ausgebildet.

Weiterhin kann die jeweilige Verschlussanlagefläche 8.1 und/oder 10.1 auch so ausgebildet sein, dass sie sich relativ zum Rotor 13 so bewegt bzw. um die Maschinenachse MA so umläuft, dass die oben erwähnte Abwälzbewegung der jeweils untersten Verschlüsse 2 und damit die gegenläufige Bewegung der Verschlüsse 2 in den Verschlussaufnahmen 14 erhalten werden.

### Bezugszeichenliste

- 1: Vorrichtung
- 2: Verschluss
- 3: Gehäuse
- 3.1 - 3.3: Gehäuseabschnitt
- 4: Inspektionsdeckel
- 5: obere Gehäusewand
- 6: Gehäusewand
- 7: Inspektionsfenster
- 8: Zwischenboden
- 8.1: Verschlussanlagefläche
- 9: Gehäusewand
- 10: untere Gehäusewand
- 10.1: Verschlussanlagefläche
- 11, 12: Behandlungszone
- 13: Rotor
- 14: Verschlussaufnahme
- 15: Halterung
- 16: Tragelement
- 17: Welle
- 18, 19: Lager
- 20, 21: Antrieb
- 22: Verschlussaufgabe
- 23, 24: Förderstrecke
- 25, 26: Verschlussführung
- 27: Behandlungsstrecke
- 28: Verschlussführung
- 29, 30: Übergabeposition
- 31: Kammer der Behandlungsstrecke 27
- 32: Einlass für Sterilisiermittel
- 33: Heizung
- 34: Umluftgebläse
- 35: Luftleitblech
- 36: Sterillufteintritt
- 37: Sterilluftaustritt
- A: Drehbewegung des Rotors 13
- B: Abwälz- oder Drehbewegung der Verschlüsse 2
- MA: vertikale Maschinenachse

## Patentansprüche

1. Vorrichtung zum Sterilisieren von Verschlüssen (2) zum Verschließen von Flaschen oder Behältern mit einem Transportsystem mit Verschlussaufnahmen (14) zur Aufnahme einer Vielzahl von Verschlüssen (2) zum Bewegen der Verschlüsse (2) durch wenigstens drei Behandlungszonen (11, 12, 27), von denen wenigstens eine erste Behandlungszone (11) zum Vorerwärmen der Verschlüsse (2), wenigstens eine zweite Behandlungszone (27) zum Beaufschlagen der Verschlüsse (2) mit einem Sterilisiermittel, und wenigstens eine dritte Behandlungszone (12) zum Trocknen der Verschlüsse (2) in einem sterilen gas- und/oder dampfförmigen Medium dienen,
**dadurch gekennzeichnet,**
**dass** das Transportsystem in der wenigstens einen ersten und dritten Behandlungszone (11, 12) jeweils wenigstens einen um eine Maschinenachse (MA) umlaufend antreibbaren Rotor (13) mit einer Vielzahl von am Umfang dieses Rotors (13) gebildeten Verschlussaufnahmen (14) in Form von parallel zur Maschinenachse (MA) verlaufenden, stabförmigen Verschlussführungsschienen aufweist, mit denen die Verschlüsse (2) durch die erste Behandlungszone (11) von einer dortigen Verschlussaufgabe (24) an eine erste Verschlussübergabeposition (29) und durch die dritte Behandlungszone (12) von einer dortigen zweiten Verschlussübergabeposition (30) an eine Verschlussabgabe (24) bewegt werden, und dass die zweite Behandlungszone (27) an wenigstens einer sich zwischen der ersten und der zweiten Übergabeposition (29, 30) erstreckenden Verschlussführung (28) für die Verschlüsse (2) ausgebildet ist, dass die e Verschlussführung (28) zur Weiterleitung der an die erste Verschlussübergabeposition (29) geförderten Verschlüsse (2) an wenigstens eine an der zweiten Verschlussübergabeposition (30) bereitstehende Verschlussaufnahme (14) des Rotors (13) In der dritten Behandlungszone (12) ausgebildet ist.

2. Vorrichtung nach Anspruch 1, **dadurch gekennzeichnet, dass** wenigstens ein Rotor (13) hohltrommel- oder hohlzylinderartig mit einem von der Vielzahl der Verschlussaufnahmen (14) gebildeten käflgartigen Trommelmantel ausgeführt ist.

3. Vorrichtung nach einem der vorhergehenden Ansprüche, **dadurch gekennzeichnet, dass** die Verschlussaufnahmen (14) jeweils käfigartig mit sich in Längsrichtung der jeweiligen Verschlussaufnahme erstreckenden stab- oder stangenförmigen Führungsschienen ausgebildet ist, zwischen denen die Verschlüsse (2) aufgenommen sind.

4. Vorrichtung nach einem der vorhergehenden Ansprüche, **dadurch gekennzeichnet, dass** die Rotoren (13) mit ihren Maschinenachsen (MA) in vertikaler Richtung orientiert sind.

5. Vorrichtung nach einem der vorhergehenden Ansprüche, **dadurch gekennzeichnet, dass** die Verschlussaufnahmen (14) mit ihrer Längserstreckung in Richtung der Maschinenachse (MA), orientiert sind.

6. Vorrichtung nach einem der vorhergehenden Ansprüche, **gekennzeichnet durch** wenigstens einen Antrieb (20, 21) für einen schrittweisen oder getackteten Antrieb der Rotoren (13) oder für jeden Rotor einen eigenständigen Antrieb (20, 21).

7. Vorrichtung nach einem der vorhergehenden Ansprüche, **gekennzeichnet durch** wenigstens eine Heizeinrichtung (33) oder eine Heizeinrichtung (33) mit Gebläse (34) zur Erzeugung einer Strömung eines heißen, gas- und/oder dampfförmigen Mediums zum Erwärmen der Verschlüsse (2) und einer die Verschlussaufnahmen (14) durchströmenden Strömung des heißen gas- und/oder dampfförmigen Mediums.

8. Vorrichtung nach einem der vorhergehenden Ansprüche, **dadurch gekennzeichnet, dass** an der dritten Behandlungszone (12) wenigstens ein Eintritt (36) sowie ein Austritt (37) für das sterile gas- und/oder dampfförmige Medium vorgesehen sind.

9. Vorrichtung nach einem der vorhergehenden Ansprüche, **gekennzeichnet durch** zusätzliche, steuerbare Verschlussauslässe (25, 26) zum Entfernen von Verschlossen (2) aus der ersten und/oder dritten Behandlungszone (11, 12) oder aus den Verschlussaufnahmen (14) der Rotoren (13) dieser Behandlungszonen und/oder **durch** Mittel zur Beaufschlagung der Behandlungszonen (11, 12, 27) mit einem Überdruck.

10. Vorrichtung nach einem der vorhergehenden Ansproche, **dadurch gekennzeichnet, dass** die Behandlungszonen (11, 12, 27) in einem Vorrichtungsgehäuse (3) ausgebildet sind, und dass zumindest eine die erste und/oder dritte Behandlungszone von einer Umgebung trennende Gehäusewand (6) mit wenigstens einem großflächigen, durch eine Verglasung dicht verschlossenen Inspektionsfenster (7) versehen ist.

11. Vorrichtung nach einem der vorhergehenden Ansprüche, **dadurch gekennzeichnet, dass** einem unteren offenen Ende der Verschlussaufnahmen (14) gegenüber liegend wenigstens eine mit dem Rotor (13) nicht mit bewegte oder relativ zum Rotor (13) bewegte Verschlussanlagefläche (8.1, 10.1) vorgesehen ist, auf der sich der dieser Fläche benachbarte Verschluss (2) bel umlaufenden Rotor (13) zur Erzeugung einer von Verschluss zu Verschluss übertragenen Verschluss-Drehbewegung (B) in der jeweiligen Versohlussaufnahme (14) abwälzt.

## Claims

1. Device for sterilizing closures (2) for closing bottles or similar containers, with a transport system with closure accommodation points (14) for accommodating a plurality of closures (2), for moving the closures through at least three treatment zones (11, 12, 27), of which at least one first treatment (11) is used for preheating the closures (2), at least one second treatment zone (27) is used for supplying the closures (2) with a sterilizing agent, and at least one third treatment zone (12) is used for drying the closures (2) in a sterile gas and/or vaporous medium,
**characterised in that** the transport system in the at least one first and third treatment zone (11, 12) is formed in each case with at least one rotor (13) capable of being driven about a machine axis (MA), with a plurality of closure accommodation points (14) formed at the circumference of this rotor (13) in the form of closure guide rails in rod shape running parallel to the machine axis (MA), with which the closures (2) are moved through the first treatment zone (11) from a first closure issue point (24) located there to a first closure handover position (29), and through the third treatment zone (12) from a second closure handover position (30) located there to a closure outlet (24), and that the second treatment zone (27) is formed at at least one closure guide (28) extending between the first and the second handover position (29, 30) for the closures (2), that the closure guide (2B) is formed for the further conducting of the closures (2) conveyed to the first closure handover position (29) at the at least one closure accommodation point (14) of the rotor (13) provided at the second closure handover position (30) in the third treatment zone (12).

2. Device according to claim 1, **characterised in that** at least one rotor (13) is designed in the form of a a hollow drum or hollow cylinder, with a cage-like drum casing formed from the plurality of closure accommodation points (14).

3. Device according to any one of the preceding claims, **characterised in that** the closure accommodation points (14) are in each case formed in the form of a cage, with guide rails in the form of bars or rods extending in the longitudinal direction of the respective closure mounting point, between which the closures (2) are accommodated.

4. Device according to any one of the preceding claims, **characterised in that** the rotors (13) are oriented with their machine axes (MA) in the vertical direction.

5. Device according to any one of the preceding claims, **characterised in that** the closure accommodation points (14) are oriented with their longitudinal extension in the direction of the axis (MA).

6. Device according to any one of the preceding claims, **characterised by** at least one drive unit (20, 21) for a stepped or pulsed drive of the rotors (13) or one individual drive unit (20, 21) for each rotor.

7. Device according to any one of the preceding claims, **characterised by** at least one heating device (33) or a heating device (33) with fans (34) for creating a current flow of a hot gas and/or vaporous medium for heating the closures (2) and a flow of the hot gas and/or vaporous medium through the closure accommodation points (14).

8. Device according to any one of the preceding claims, **characterised in that**, at the third treatment zone (12), at least one inlet (36) is provided, as well as an outlet (37) for the sterile gas and/or vaporous medium.

9. Device according to any one of the preceding claims, **characterised by** additional controllable closure outlets (25, 26) for removing closures (2) from the first and/or second treatment zones (11, 12) or from the closure accommodation points (14) of the rotors (13) of these treatment zones and/or by means for imposing an overpressure on the treatment zones (11,12, 27).

10. Device according to any one of the preceding claims, **characterised in that** the treatment zones (11, 12, 27) are formed in a device housing (3), and that at least one housing wall (6) is provided, separating the first and/or third treatment zone from the surrounding area, with at least one large-surface inspection window (7) tight sealed by a glazing element.

11. Device according to any one of the preceding claims, **characterised in that** at least one closure support surface (8.1, 10.1) is provided, lying opposite a lower open end of the closure accommodation points (14), which is not moved with the rotor (13) or is moved relative to the rotor (13), on which the closure (2) adjacent to this surface rolls, when the rotor (13) rotates, in order to produce a rotational movement (B) for the closures in the respective closure accommodation point (14) which is transferred from closure to closure.

## Revendications

1. Dispositif destiné à stériliser des éléments de fermeture (2) pour fermer des bouteilles ou des récipients, comportant un système de transport doté de réceptacles d'éléments de fermeture (14) pour recevoir une multiplicité d'éléments de fermeture (2) pour déplacer les éléments de fermeture (2) dans au moins trois zones de traitement (11, 12, 27) parmi lesquelles au moins une première zone de traitement (11) sert à préchauffer des éléments de fermeture (2), au moins une deuxième zone de traitement (27) sert à charger les éléments de fermeture (2) avec un agent de stérilisation et au moins une troisième zone de traitement (12) sert à sécher les éléments de fermeture (2) dans un milieu stérile sous forme de gaz et/ou de vapeur, **caractérisé en ce que** le système de transport dans les au moins une première et troisième zones de traitement (11, 12) présente respectivement au moins un rotor (13) qui peut être entraîné de manière rotative autour d'un axe de machine (MA), comportant une multiplicité de réceptacles d'éléments de fermeture (14) formés sur le pourtour de ce rotor (13) sous la forme de rails de guidage d'éléments de fermeture sous forme de baguettes évoluant parallèlement à l'axe de la machine (MA), avec lesquels les éléments de fermeture (2) sont déplacés dans la première zone de traitement (11) d'une charge d'élément de fermeture (24) à ce niveau à une première position de transmission d'élément de fermeture (29) et dans la troisième zone de traitement (12) d'une deuxième position de transmission d'élément de fermeture (30) à ce niveau à une restitution d'élément de fermeture (24) et **en ce que** la deuxième zone de traitement (27) est formée sur au moins un guidage d'élément de fermeture (28) s'étendant entre la première et la deuxième position de transmission (29, 30) pour les éléments de fermeture (2), **en ce que** le guidage d'élément de fermeture (28) est conformé pour l'acheminement des éléments de fermeture (2) transportés à la première position de transmission d'élément de fermeture (29) sur au moins un réceptacle d'élément de fermeture (14) du rotor (13) disponible sur la deuxième position de transmission d'élément de fermeture (30) dans la troisième zone de traitement (12).

2. Dispositif selon la revendication 1, **caractérisé en ce qu'**au moins un rotor (13) de type à tambour creux ou à cylindre creux est réalisé avec une paroi de tambour de type cage formée de la multiplicité des réceptacles d'éléments de fermeture (14).

3. Dispositif selon l'une des revendications précédentes, **caractérisé en ce que** les réceptacles d'éléments de fermeture (14) respectivement en forme de cage sont formés avec des rails de guidage en forme de baguette ou de barre s'étendant dans la direction longitudinale des réceptacles d'éléments de fermeture respectifs, entre lesquels les éléments de fermeture (2) sont reçus.

4. Dispositif selon l'une des revendications précédentes, **caractérisé en ce que** les rotors (13) sont orientés avec leurs axes de machine (MA) en direction verticale.

5. Dispositif selon l'une des revendications précédentes, **caractérisé en ce que** les réceptacles d'éléments de fermeture (14) sont orientés avec leur dimension longitudinale en direction de l'axe de machine (MA).

6. Dispositif selon l'une des revendications précédentes, **caractérisé par** au moins un entraînement (20, 21) pour un entraînement par paliers ou de façon cadencée des rotors (13) ou, pour chaque rotor, un entraînement autonome (20, 21).

7. Dispositif selon l'une des revendications précédentes, **caractérisé par** au moins un système de chauffage (33) ou un système de chauffage (33) avec des ventilateurs (34) pour générer un courant d'un milieu chaud sous forme de gaz et/ou de vapeur pour chauffer les éléments de fermeture (2) et un courant parcourant les réceptacles d'éléments de fermeture (14) du milieu chaud sous forme de gaz et/ou de vapeur.

8. Dispositif selon l'une des revendications précédentes, **caractérisé en ce que**, sur la troisième zone de traitement (12), sont prévues au moins une entrée (36) et une sortie (37) pour le milieu stérile sous forme de gaz et/ou de vapeur.

9. Dispositif selon l'une des revendications précédentes, **caractérisé par** des dégagements d'éléments de fermeture (25, 26) supplémentaires, pouvant être commandés, pour retirer des éléments de fermeture (2) de la première et/ou de la troisième zone de traitement (11, 12) ou des réceptacles d'éléments de fermeture (14) des rotors (13) de ces zones de traitement et/ou **caractérisé par** des moyens d'apport d'une surpression aux zones de traitement (11, 12, 27).

10. Dispositif selon l'une des revendications précédentes, **caractérisé en ce que** les zones de traitement (11, 12, 27) sont formées dans un boîtier de dispositif (3) et **en ce qu'**au moins une paroi de boîtier (6) séparant la première et/ou la troisième zone de traitement d'un environnement est dotée d'au moins une large fenêtre d'inspection (7) scellée hermétiquement par une vitrification.

11. Dispositif selon l'une des revendications précédentes, **caractérisé en ce que**, d'un côté opposé d'une extrémité inférieure ouverte des réceptacles d'éléments de fermeture (14), est prévue au moins une surface d'appui d'éléments de fermeture (8.1, 10.1) qui n'est pas déplacée en même temps que le rotor (13) ou est déplacée par rapport au rotor (13), sur lequel l'élément de fermeture (2) voisin de cette surface roule alors que le rotor tourne (13) pour générer un mouvement rotatif d'élément de fermeture (8) transmis d'un élément de fermeture à l'autre dans les réceptacles d'élément de fermeture respectifs (14).
